# EUROPÄISCHE PATENTANMELDUNG

(11) **EP 3 144 291 A1**
(43) Veröffentlichungstag der Anmeldung: **22.03.2017**
(21) Anmeldenummer: 15185441.1
(22) Anmeldetag: 16.09.2015
(51) Int. Cl.: C07C 67/39, C07C 67/44, C07C 51/09, C07C 69/54, C07C 57/04

(54) **SYNTHESE VON METHACRYLSÄURE AUS AUF METHACROLEIN BASIERENDEN ALKYLMETHACRYLAT**

(71) Anmelder: Evonik Röhm GmbH, 64293 Darmstadt (DE)
(72) Erfinder: KRILL, Steffen, 64367 Mühltal (DE); GRÖMPING, Matthias, 64295 Darmstadt (DE); LYGIN, Alexander, 64347 Griesheim (DE)

(57) **Zusammenfassung**

Die vorliegende Erfindung betrifft ein Verfahren zur Herstellung von Alkylmethacrylaten, insbesondere MMA und von Methacrylsäure auf Basis von Methacrolein, welches in einer zweiten Verfahrensstufe oxidativ verestert wurde. Methacrolein ist dabei grundsätzlich aus C₂- und C₄-Bausteinen zugänglich. Das vorliegende Verfahren hat dabei den Vorteil, dass das Alkylmethacrylat und die Methacrylsäure einfach, in hohen Ausbeuten und hohen Reinheiten, wahlweise als Gemisch oder als isolierte Produktströme gewonnen werden können. Dabei weist das erfindungsgemäße Verfahren insbesondere den großen Vorteil auf, dass insbesondere das Verhältnis der gewünschten Produkte Methacrylsäure und Alkylmethacrylat, insbesondere MMA in einem großen Bereich frei eingestellt und durch verfahrenstechnische Maßnahmen und Prozessparameter variiert werden kann.

## Beschreibung

### Gebiet der Erfindung

Die vorliegende Erfindung betrifft ein Verfahren zur Herstellung von Alkylmethacrylaten, insbesondere MMA und von Methacrylsäure auf Basis von Methacrolein, welches zu einem Alkylmethacrylat oxidativ verestert wird. Methacrolein ist dabei grundsätzlich aus C₂- und C₄-Bausteinen zugänglich. Das vorliegende Verfahren hat dabei den Vorteil, dass das Alkylmethacrylat und die Methacrylsäure einfach, in hohen Ausbeuten und hohen Reinheiten, wahlweise als Gemisch oder als isolierte Produktströme gewonnen werden können. Dabei weist das erfindungsgemäße Verfahren insbesondere den großen Vorteil auf, dass insbesondere das Verhältnis der gewünschten Produkte Methacrylsäure und Alkylmethacrylat, insbesondere MMA in einem großen Bereich frei eingestellt und durch verfahrenstechnische Maßnahmen und Prozessparameter variiert werden kann.

### Stand der Technik

Aus dem Stand der Technik ist eine Vielzahl von Verfahren zur Herstellung von Methacrylsäure bekannt.
Eine übliche Verfahrensweise besteht in der kontrollierten Oxidation von Kohlenwasserstoffgasen, beispielsweise Butylen. Nachteilig an diesen Verfahren sind die insgesamt gesehen eher geringen Ausbeuten, die hierdurch erhalten werden.
Darüber hinaus kann Methacrylsäure durch die Umsetzung von Methacrylamid mit Wasser erhalten werden. Dieses Verfahren wird insbesondere in US 7,253,307 beschrieben. Gemäß dieser Druckschrift kann die Umsetzung des Methacrylamids mit Wasser in einem Rührkesselreaktor oder einem Röhrenreaktor erfolgen. Vorzugsweise wird die Reaktion bei einem Druck im Bereich von 3,65 bis 7,70 bar und einer Temperatur im Bereich von 50 bis 210 °C durchgeführt.
Die in US 7,253,307 beschriebenen Verfahren zur Herstellung von Methacrylsäure führen bereits zu guten Ausbeuten bei einer hohen Reinheit. Allerdings stellt Methacrylsäure ein wichtiges Erzeugnis der chemischen Industrie dar, welches als Ausgangsstoff für viele wichtige Produkte dient. Daher sind eine maximale Ausbeute und eine besonders hohe Reinheit bei geringen Herstellungskosten wesentlich für den wirtschaftlichen Erfolg eines solchen Herstellungsprozesses. Schon relativ kleine Verbesserungen hinsichtlich der Ausbeuten, der Standzeiten der Anlagen oder ähnlicher Verfahrensmerkmale führen zu einem bedeutenden Fortschritt hinsichtlich der Abfallmengen und der Herstellkosten. Methacrylsäureamid selbst wird üblicherweise nach dem Acetoncyanhydrin (kurz ACH) - Sulfo Verfahren auf Basis von Blausäure und Aceton in Gegenwart eines großen Überschusses an Schwefelsäure hergestellt. Bei dem Verfahren werden große Mengen an Abfallschwefelsäure bzw. organisch verunreinigter Ammoniumhydrogensulfat Lösung erzeugt. Diese können nur mit großem energetischen Aufwand zu Schwefelsäure aufgearbeitet werden.

Ebenso kann α-Hydroxyisobuttersäure (HIBS) als Ausgangsstoff zur Herstellung von Methacrylsäure dienen. Ein derartiges Verfahren wird zum Beispiel in US 3,487,101 beschrieben, wo die Herstellung diverser Methacrylsäurederivate, insbesondere Methacrylsäure und Methacrylsäureester, ausgehend von 2-Hydroxyisobuttersäure (HIBS) in der Flüssigphase, dadurch gekennzeichnet ist, dass die Umsetzung von HIBS zu Methacrylsäure in Gegenwart eines gelösten basischen Katalysators bei hohen Temperaturen zwischen 180- 320 °C in Gegenwart hochsiedender Ester (z.b. Phthalsäuredimethylester) und cyclischen Anhydriden (z.B. Phthalsäureanhydrid) durchgeführt wird. Laut Patent werden bei HIBS Umsätzen von > 90 % MAS-Selektivitäten um 98 % erreicht. Über die Langzeitstabilität der flüssigen Katalysatorlösung insbesondere der Stabilität des eingesetzten Anhydrids unter Reaktionsbedingungen werden keine Angaben gemacht.

RU 89631 betrifft ein Verfahren zur Herstellung von Methacrylsäure ausgehend von HIBS durch Wasserabspaltung in flüssiger Phase, dadurch gekennzeichnet, dass die Reaktion in Abwesenheit eines Katalysators mit einer wässrigen Lösung von HIBS (bis 62 Gew.-% HIBS in Wasser) unter Druck bei hohen Temperaturen 200°C - 240°C durchgeführt wird.
Intensiv untersucht wurde darüber hinaus die Verwendung von Propen als Basisrohstoff, wobei man über die Stufen Hydrocarbonylierung zur Isobuttersäure und dehydrierender Oxidation in moderaten Ausbeuten zur Methacrylsäure gelangt. Dabei handelt es sich um eine Umsetzung von Propen mit Kohlenmonoxid, Flusssäure oder konzentrierter Schwefelsäure und die anschließende Hydrolyse der Zwischenprodukte in Gegenwart von Wasser. Das Verfahren findet keine Anwendung in der Produktion.

Weiterhin ist es bekannt, Propanal, welches in technischen Prozessen ausgehend von Ethylen und C-1 Bausteinen wie Kohlenmonoxid zugänglich ist, als Basisrohstoff einzusetzen. In diesen Prozessen wird in einer aldolisierenden Reaktion mit Formaldehyd unter Dehydratisierung der in situ entstehenden β-Hydroxycarbonylverbindung zur entsprechenden α, β-ungesättigten Verbindung, dem Methacrolein, umgesetzt.
Eine Übersicht über die gängigen Verfahren zur Herstellung der Methacrylsäure und deren Ester findet sich in der Literatur wie z.B. Weissermel, Arpe "Industrielle organische Chemie", VCH, Weinheim 1994, 4. Auflage, S.305 ff oder Kirk Othmer "Encyclopedia of Chemical Technology", 3. Ausgabe, Vol. 15, Seite 357.

EP 0 487 853 beschreibt die Herstellung von Methacrylsäure ausgehend von Acetoncyanhydrin (ACH), dadurch gekennzeichnet, dass man im ersten Schritt ACH mit Wasser bei moderaten Temperaturen in Gegenwart eines heterogenen Hydrolysekatalysators umsetzt und man im zweiten Schritt α-Hydroxyisobuttersäureamid mit Methylformiat oder Methanol/Kohlenmonoxid unter Entstehung von Formamid und Hydroxyisobuttersäuremethylester (HIBSM) umsetzt, und man im dritten Schritt HIBSM in Gegenwart eines heterogenen Ionenaustauschers mit Wasser zu HIBS verseift, und man im vierten Schritt HIBS dehydratisiert, indem man in flüssiger Phase bei hohen Temperaturen in Gegenwart eines löslichen Alkalisalzes reagieren lässt. Die Methacrylsäure-Herstellung ex HIBS wird bei hohen Umsätzen um 99 % mit mehr oder weniger quantitativen Selektivitäten beschrieben. Die Vielzahl der notwendigen Reaktionsschritte und die Notwendigkeit der Zwischenisolierung einzelner Intermediate, insbesondere auch die Durchführung einzelner Prozessschritte bei erhöhtem Druck, machen das Verfahren kompliziert und damit letztlich unwirtschaftlich. Des Weiteren fällt zwingend Formamid an, wobei diese Verbindung vielfach als unerwünschtes Nebenprodukt angesehen wird, welches teuer entsorgt werden muss. In einer weiteren Variante kann Formamid zur Herstellung von HCN eingesetzt werden. Letztlich verbleiben hier aber die Nachteile der vielstufigen Reaktion mit aufwändigen Kreisläufen, was wiederum zu einem hohen energetischen Aufwand und insbesondere zu einem hohen spezifischem Dampfverbrauch führt.

Aus der EP 2 714 640 ist schließlich ein Verfahren bekannt, bei dem die Methacrylsäure durch Hydrolyse von auf ACH basierenden Methylmethacrylat (MMA) gewonnen wird. Dabei wird das ACH zunächst zu Methacrylamid umgesetzt und dieses anschließend mit Methanol zu MMA verestert. Damit besteht bei diesem Verfahren zur MMA-Herstellung im Gegensatz zu anderen Verfahren keine Möglichkeit, Methacrylsäure in Form eines Zwischenproduktes auszuschleusen. Weiterhin muss das zugeführte Wasser erhitzt werden. Dies ist insbesondere aus energetischer Sicht aufgrund der hohen Wärmekapazität des Wassers und dem damit verbundenen relevanten Energieeintrag nachteilig.

Eine solche Hydrolyse ist jedoch nur für dieses spezifische ACH-Verfahren bekannt. Es gibt mittlerweile jedoch effizientere Alternativen zur Herstellung von MMA, die gleichsam keine Methacrylsäure als Nebenprodukt aufweisen. So ist in WO 2014/170223 ein sehr effizientes Verfahren beschrieben, bei dem aus C₂-Fraktionen in einer ersten Stufe Propionaldehyd gewonnen wird und dieses in einer zweiten Stufe mit Formaldehyd zu Methacrolein (MAL) umgesetzt wird. Dieses MAL wiederum kann darauf in Anwesenheit von speziellen Metall- oder Metalloxid-Katalysatoren und von Methanol oxidativ zu MMA verestert werden. Dieses Verfahren zeichnet sich gegenüber sämtlichen anderen Verfahren durch eine besonders hohe Ausbeute und besonders gute Selektivitäten aus. Jedoch hat es den Nachteil, dass man Methacrylsäure gleichsam nicht oder in einem nur sehr geringen Umfang als Nebenprodukt erhält.
Eine Übertragung des aus EP 2 714 640 bekannten Hydrolyseverfahrens auf diese MMA-Herstellung ist nicht bekannt, zumal dieses Hydrolyseverfahren den Einsatz von gereinigtem MMA beschreibt und nicht von Zwischenproduktmischungen, die MMA neben den in diesem Verfahren spezifisch erzeugten Nebenprodukten enthält.

Wichtig in diesem Zusammenhang ist auch, dass je nach eingesetzter Technologie und Rohstoffbasis jeweils andere problematische Nebenprodukte entstehen, sodass ein großer Unterschied besteht, ob das MMA aus einem ACH-Sulfo Verfahren erzeugt wird oder beispielsweise basierend auf einem C-2 Rohstoff (Ethylen) wie in WO 2014/170223 beschrieben, eingesetzt wird.

Eine einfache Integration einer MMA zu Methacrylsäure (MAS) Hydrolyse, wie in EP 2 714 640 beschrieben, in ein MMA Herstellungsverfahren ausgehend von Methacrolein würde bedeuten, dass viele zusätzliche Trennungsschritte eingebaut werden müssten, was wiederum hohe zusätzlichen Investitionskosten hervorrufen und insgesamt keine Synergieeffekte zwischen zwei Verfahren aufweisen würde.

Auch ist diese nicht einfach durchzuführen, da das auf C₂ basierende MMA gemäß der WO 2014/170223 insbesondere in den Aufarbeitungsschritten ein gänzlich anderes NebenproduktSpektrum aufweist, welches unter Berücksichtigung hoher Ausbeuten und Selektivitäten berücksichtigt werden muss. So ist bekannt, dass das MMA, welches gemäß WO 2014/170223 hergestellt wurde, trotz Aufreinigung zu Gelbfärbungen neigen kann. Weiterhin enthält dieses Roh-MMA insbesondere höhere Konzentrationen an Methacrylsäure, an 1,1-Dimethoxyisobuten oder an Michael-Produkten des MAL und dimeres MAL, sowie deren Folgeprodukte aus der oxidativen Veresterung, wie beispielsweise die entsprechenden Säuren und Methylester. Insbesondere das dimere MAL führt damit zu relevanten Mengen an Methylester und/oder Säure im MMA. Wenn man diese ungewünschten Nebenprodukte nun vermeiden möchte, ist es nötig, das MMA vor der Hydrolyse zu reinigen. Dies erfolgt primär mittels einer Destillation. Dabei werden jedoch die gleichfalls als Nebenprodukt der oxidativen Veresterung anfallende Methacrylsäure abgetrennt und gehen damit der Ausbeute verloren.

In der EP 0 092 097 schließlich wird ein auf C2-Basis, über das Intermediat Methacrolein (MAL) geführtes Verfahren offenbart. Hier wird Methacrylsäure direkt in einem an die MAL-Synthese anschließenden Gasphasenschritt erzeugt und kann optional ausgeschleust, aufgearbeitet und isoliert werden. Das eigentliche Ziel des Verfahrens ist die Veresterung mit Methanol zu MMA in einer dritten Stufe. Somit ist dieses Verfahren zwar geeignet Methacrylsäure neben MMA herzustellen, die erreichbaren Ausbeuten sind aber durch den nicht befriedigenden Schritt von MAL zu MAS in der Gasphase limitiert, was sich unterm Strich in der Wirtschaftlichkeit des Verfahrens negativ auswirkt. Nachteilig an diesem seitens der Rohstoffbasis effizienten Verfahren ist insbesondere die Folgeumsetzung in der Gasphase an einer Heteropolysäure. Hierbei werden nur Teilumsätze des MAL erreicht und die Ausbeute beträgt auch nach Patentliteratur maximal 80 bis 85% an Methacrylsäure. Selbst in der Gasphase hat die Anwesenheit von Nebenprodukten aus der MAL Herstellung aus Propionaldehyd wie dimeres Methacrolein und Pentenale negative Auswirkungen, sodass diese Nebenprodukte strikt limitiert werden müssen.

Viele der aufgeführten Verfahren, insbesondere zur Hydrolyse, weisen zudem den Nachteil auf, dass Wasser in das System eingebracht werden muss. Dieses muss jedoch vor der Zugabe oder im Reaktor auf die Reaktionstemperatur aufgeheizt werden.

Zusammenfassend bleibt festzuhalten, dass es bis dato keine wirtschaftliche und technisch naheliegende oder technisch einfache Lösung gibt, um z.B. basierend auf Methacrolein als Ausgangsbasis und Rohstoff, MMA neben Methacrylsäure herzustellen und hierbei insbesondere das Produktverhältnis der beiden Wertstoffe in einem breiten Verhältnis bei gleichzeitig guten Gesamtausbeuten und letztlich optimal wirtschaftlich herzustellen.

### Aufgabe

Aufgabe der Erfindung ist es daher ein neues effizientes und wirtschaftlich attraktives Verfahren zur Herstellung von Alkylmethacrylaten, insbesondere von MMA und gleichzeitig von Methacrylsäure ausgehend von C₂-Bausteinen zur Verfügung zu stellen.

Insbesondere soll ein Verfahren zur Verfügung gestellt werden, mittels dem die Problematik variierender Marktbedürfnissen bzw. Marktanforderungen an Methacrylsäure und MMA Rechnung getragen wird und dazu beide Wertprodukte in einem nahezu frei wählbaren Bereich durch die Wahl entsprechender verfahrenstechnischer Parameter erzeugt werden können.

Weiterhin soll das neue Verfahren mit einem geringeren Energieverbrauch auskommen, sowie höhere Gesamtausbeuten an Methacrylsäure bzw. Alkylmethacrylaten, insbesondere MMA ermöglichen.

Darüber hinaus sollen die mittels dem Verfahren hergestellten Alkylmethacrylate und Methacrylsäure geringe Farbzahlen aufweisen.

Insbesondere war es Aufgabe der vorliegenden Erfindung, die genannten Nachteile anderer Verfahren zu vermeiden, insbesondere soll das Verhältnis aus Alkylmethacrylaten und Methacrylsäure im Produkt durch die Prozessführung und die Prozessparameter steuerbar sein.

Weiterhin bestand die Aufgabe, ein Verfahren zur Herstellung von Methacrylsäure durch Hydrolyse von MMA zur Verfügung zu stellen, bei dem kein zusätzliches Wasser in das System gegeben werden muss.

Weiter nicht explizit genannte Aufgaben können sich aus der Beschreibung oder den Ansprüchen ergeben.

### Lösung

Gelöst wurden diese Aufgaben durch Bereitstellung eines neuartigen Verfahrens zur Herstellung von Methacrylsäure, welches folgende Prozessschritte aufweist:
a) Synthese von Methacrolein in einem Reaktor I,
b) Oxidative Veresterung von Methacrolein mit einem Alkohol und Sauerstoff zu einem Alkylmethacrylat in einem Reaktor II und
e) Umsetzung mindestens eines Teils des Alkylmethacrylats mit Wasser zu Methacrylsäure in einem Reaktor III.

Insbesondere bevorzugt sind erfindungsgemäß Verfahren, die mehr detailliert folgende Prozessschritte aufweisen:
a) Synthese von Methacrolein in einem Reaktor I,
b) oxidative Veresterung von Methacrolein mit einem Alkohol und Sauerstoff zu einem Alkylmethacrylat in einem Reaktor II,
c) Abtrennen des überschüssigen Methacroleins und zumindest teilweises Abtrennen des Alkohols, anschließende optionale Behandlung mit einer Säure und optionale Phasentrennung der Alkylmethacrylat enthaltenden Zusammensetzung,
d) Trennen der Alkylmethacrylat und optional Methacrylsäure enthaltenden Zusammensetzung unter Zuführen von Wasser als eine organische Phase von einer wässrigen Phase in einer Extraktion I,
e) Umsetzung mindestens eines Teils des Alkylmethacrylats mit Wasser zu Methacrylsäure in einem Reaktor III,
f) Überführen der Zusammensetzung, enthaltend Methacraylsäure und Alkylmethacrylate aus Prozessschritt e) in die Extraktion I aus Prozessschritt d) und
g) optionale Trennung der Methacrylsäure von dem Alkylmethacrylat in einer Trennstufe M.

Das erfindungsgemäße Verfahren kann auf sämtliche auf Methacrolein und anschließende oxidative Veresterung dieses Methacroleins basierende Verfahren angewendet werden, Dabei kann das Methacrolein auf Basis von C2 oder C4 hergestellt worden sein. Insbesondere kann das erfinderische Verfahren auf die Kombinationen aus einem C2-basierten Verfahren zur Herstellung von Methacrolein und eine anschließende oxidative Veresterung zu einem Alkylmethacrylat angewendet werden. Dies betrifft insbesondere die in DE 3 213 681, US 4,408,079, CN 1 038 461 04 und in der europäischen Patentanmeldung mit dem Anmeldeaktenzeichen 14185345.7 beschriebenen Verfahren.

Ein besonderer Aspekt der vorliegenden Erfindung ist die Möglichkeit, die Wasserströme innerhalb des Gesamtprozesses besonders energiesparend gestalten zu können und die Nebenproduktenbildung in einzelnen Prozessschritten so einzustellen, dass diese sich nicht gegenseitig stören. So wird Wasser in verschiedenen Stufen dem Prozess zugeführt. Das betrifft zunächst Stufe a), in der dem Reaktor I (6) z.B. mit Formaldehyd (1), der Base (3) und/oder der Säure (4) Wasser zugeführt wird. Der genaue Wassergehalt im Methacrolein-Produktaustrag nach Isolierung (8), welcher relevant bezüglich der Bildung von Nebenprodukten ist, kann dabei in Abhängigkeit von den weiteren Prozess- und Isolierungsparametern, wie Temperatur, Druck, Katalysatorkonzentration oder Verweilzeit, t beeinflusst werden und ist vom Fachmann auf Basis des bekannten Standes der Technik für diese Prozessstufe zu optimieren.
Das unter optimalen Prozessparameter hergestellte Methacrolein, wie z.B. in der europäischen Patentanmeldung mit dem Anmeldeaktenzeichen 14185345.7 beschrieben, wird direkt oder über (12) in den Reaktor II (9) eingeleitet, wo der Wassergehalt allein schon durch das bei der Reaktion entstehende Wasser steigt. Weiterhin können hier auch durch die zugegebene Base und eventuell den zugegebenen Alkohol weitere Wasseranteil dazukommen. Insbesondere in dieser Stufe ist auf den genauen Wasseranteil zu achten, da hier ganz besonders die Bildung von Nebenprodukten von diesem abhängen kann. Eine weitere Wasserzugabe erfolgt darauf eventuell durch die bevorzugte Zugabe einer Säure (14).

Eine genauso bevorzugte Variante der vorliegenden Erfindung ist es, den in Prozessschritt e) gebildete Alkohol ganz oder teilweise abzutrennen und in Prozessschritt b) dem Reaktor II zur oxidativen Veresterung ganz oder teilweise wieder zuzuführen.

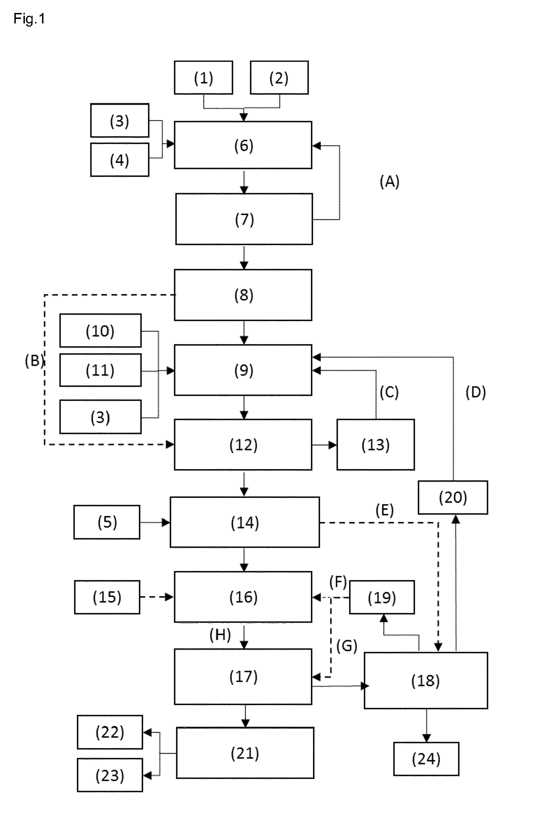

Die Abtrennung des Wassers aus der Reaktionsmischung erfolgt dann zunächst in dem Prozessschritt c), z.B. in einer optionalen Phasentrennung, und Prozessschritt d), der Extraktion I (17), zusammen mit dem vorliegenden Alkohol. Bevorzugt wird diese Mischung in einer weiteren Kolonne (18) in Alkohol (20) und Wasser (19) getrennt. Dieses Wasser hat je nach Druck noch eine Temperatur von ca. 100 °C und kann einerseits zusammen mit dem Kolonnensumpf entsorgt werden. Bevorzugt jedoch - und hier liegt ein weiterer Vorteil der Erfindung - wird dieses Wasser (19) vollständig oder teilweise dem Reaktor III in Prozessstufe e) zugeführt. Dies hat den besonderen Vorteil, dass es nicht wie extern zugeleitetes Wasser zusätzlich erhitzt werden muss und somit der Prozess besonders wirtschaftlich und unter der Vermeidung großer Mengen Wassers zur Entsorgung durchgeführt werden kann. Dieses Recycling des zuvor zugeführten Wassers aus den Prozessschritten a) bis b), sowie des Reaktionswassers aus Prozessschritt b) ist in allen beschriebenen Ausführungsformen -wie mit den gestrichelten Linien (F) in den Figuren 1 bis 3 beispielhaft aufgezeigt - realisierbar. Alternativ oder zusätzlich können auch Teilströme dieses Wassers zurück in die Extraktion I (17) geleitet werden (G). Weiterhin muss zumindest ein Teilstrom des abgetrennten Wassers (19) einer Entsorgung zugeführt werden, da sich ansonsten innerhalb des Gesamtprozesses, genauer in den Komponenten (16), (17) und (18) Wasser anreichern würde. Dieser Teilstrom ist für den Fachmann derart einzustellen, dass der Wassergehalt in diesen Komponenten, und damit in den Prozessschritten d), e) und f) unabhängig von deren Reihenfolge, konstant bleibt.

Der Extraktion I kann optional eine weitere Phasentrennung vorgeschaltet sein. Die wässrige Phase aus dieser Phasentrennung enthält anteilig den Alkohol, Wasser und das Alkalisalz der Methacryslsäure und/oder das Alklalisalz der zugegebenen mineralischen Säure. Zum Beispiel kann diese wässrige Phase entsprechend der wässrigen Phase der nachgeschalteten Extraktion (17) weiterbehandelt werden. Die organische Phase der Phasentrennung wird in die Extraktion I weitergeleitet.

Daher ist es unabhängig von der verwendeten Ausführungsform der vorliegenden Erfindung besonders bevorzugt, wenn in Prozessschritt b) gebildetes Reaktionswasser zwischen den Prozessschritten b) und e), insbesondere in Prozessschritt d) von dem Alkylmethacrylat abgetrennt und in Prozessschritt e) dem Reaktor III zur Hydrolyse ganz oder teilweise wieder zugeführt wird. Genauso bevorzugt und ergänzend oder unabhängig von den Wasserströmen wird der abgetrennte Alkohol aus der Hydrolyse in Reaktor III der oxidativen Veresterung in Reaktor II wieder zugeführt und damit gleichsam wiederverwendet.

Überraschend wurde gefunden, dass mit dem erfindungsgemäßen Verfahren eine großtechnisch einfach realisierbare Methacrylsäuresynthese zur Verfügung gestellt werden kann. Das Verfahren zeichnet sich durch die oben genannten Vorteile sowie durch ein geringes Nebenproduktspektrum aus. Die erhaltene isolierte Methacrylsäure weist eine Reinheit von zumeist größer 99,5 % auf.

Prozessschritte a) bis c) sind dem Fachmann allgemein insbesondere basierend auf einer C2-Rohstoffbasis bekannt und können beispielsweise in WO 2014/170223 oder der internationalen Patentanmeldung mit dem Anmeldeaktenzeichen PCT/EP 2014/068171 nachgelesen werden. Das Methacrolein in Prozessschritt a) kann dabei auf Basis von C₄-Bausteinen, wie Isobuten oder tert-Butanol, oder auf Basis von C₁- und C₂-Bausteinen, insbesondere aus Propanal und Formaldehyd, wobei das Propanal wiederum aus Ethylen, Wasserstoff und Kohlenmonoxid gewonnen wird, synthetisiert werden.

Bevorzugt wird das Methacrolein in Prozessschritt a) aus Propionaldehyd und Formaldehyd über eine Mannich Kondensation hergestellt. Besonders bevorzugt wird das in Prozessschritt c) abgetrennte Methacrolein und - zumindest teilweise - der überschüssige Alkohol in den Reaktor II zurückgeführt.

Für das erfindungsgemäße Verfahren ist es sehr vorteilhaft, das bei der Methacrolein-Synthese entstehende Nebenprodukt eines cyclischen dimeren Methacroleins zu minimieren oder dieses Nebenprodukt vor der Hydrolyse aus der Reaktionsmischung zu entfernen. Das dimere Methacrolein wird in Reaktor II selektiv zum Alkylester des dimeren Methacroleins verestert und würde so in die Folgestufen gelangen. In der Hydrolyse würde dieser Ester wiederum zur freien Säure des dimeren Methacroleins hydrolisiert werden. Diese Nebenprodukte des dimeren Methacroleins, dessen Alkylesters bzw. der entsprechenden freien Säure werden in dem erfindungsgemäßen Verfahren effektiv von den Zielprodukten durch eine entsprechend gestaltete Produktaufarbeitung abgetrennt.

Insbesondere gibt es drei gleichsam bevorzugte Ausführungsformen der vorliegenden Erfindung. Diese drei Varianten unterscheiden sich insbesondere in der Stromführung und damit teilweise auch in der Reihenfolge der einzelnen Prozessschritte. Dabei ist bei allen drei Ausführungsformen der Prozess bis zu einer in Prozessschritt c) bevorzugt stattfindenden Versetzung mit einer Säure (14) und einer optionalen Phasentrennung, identisch durchführbar.

Insbesondere erfolgt der Prozess bis zu diesem Schritt ausgehend von der Synthese des Methacroleins in Reaktor I (6) aus Formaldehyd (1), Propionaldehyd (2) unter Zugabe eines aus mindestens einer Base (3) und einer Säure (4) gebildeten Katalysators. Bevorzugt erfolgt direkt nach dem Austrag des Reaktionsproduktes eine z.B. destillative Abtrennung des Katalysators (7) und die Rückführung dieses Katalysators (A) in Reaktor I (6). Die gewonnene Methacrolein-haltige Phase wird darauf, z.B. in einer Phasentrennung (8), weiter gereinigt. Dabei kann die wässrige Phase auf die Destillationskolonne (7) zurückgeführt werden. Nach der Phasentrennung (8) können optional weitere Trennungsschritte, z.B. eine zusätzliche Destillationskolonne zur weiteren MAL Reinigung, installiert werden. Anschließend wird das gereinigte Methacrolein in Reaktor II (9) zu oxidativen Veresterung unter Zuleitung von Alkohol, insbesondere Methanol (10), einer Base (3), die unterschiedlich oder identisch zur Base in Reaktor I sein kann, und Sauerstoff (11), welches optional als Luft, Reingas oder bevorzugt als Mischung mit Stickstoff zugeleitet wird. Der Austrag aus Reaktor II (9) wird anschließend derart gereinigt, dass überschüssiges Methacrolein bevorzugt als Gemisch (13), z.B. mit Alkohol isoliert und wird und in Reaktor II (9) wieder zurückgeführt wird (C). Dies kann beispielsweise in einer Destillationskolonne (12) erfolgen. In einer Variante dieses Verfahrens wird das Methacrolein optional ganz oder teilweise aus der Prozessstufe (8) direkt in die Destillation (12) geleitet, um von dort über (C) in Reaktor II (9) geleitet zu werden.

Die erste Ausführungsform ist beispielhaft in Fig.1 dargestellt. In dieser Variante wird der ein Alkylmethacrylat enthaltende Produktstrom aus Prozessschritt c) nach dem optionalen Versetzen mit einer Säure und optionaler Phasentrennung (14) zur Durchführung des Prozessschritts e) direkt in den Reaktor III (16) geleitet. In Reaktor III wird zusätzlich Wasser zugeleitet, welches in Form von Frischwasser (15) und/oder aus einem passenden Recyclingstrom (F) erfolgen kann. Gemäß Prozessschritt f) erfolgt dann die Weiterlerleitung (H) (entspricht Prozessschritt f) des Produktes aus Reaktor III (16) in die Extraktion I (17) zur Durchführung des Prozessschritts d). Die erste Phase dieser Extraktion, enthaltend das Alkylmethacrylat, insbesondere MMA und die Methacrylsäure wird zur weiteren Trennung in die Trennstufe M geleitet. Dies entspricht Prozessschritt g), aus dem ein Alkylmethacrylat, insbesondere MMA-Strom (22) und ein Methacrylsäure-haltiger Strom (23) gewonnen werden. Diese können jeweils weiteren Reinigungsstufen unterzogen werden. Insbesondere im Falle des Methacrylsäure-haltigen Stroms kann dieser auch direkt unter Vorliegen relevanter Restmengen des Alkylmethacrylats verwendet werden. Auch ist es möglich einen Mischstrom beider Produkte vor der Extraktion zu isolieren, um diesen gemeinsam zu reinigen und einer Verwendung als Mischung zuzuführen. Die überwiegend wässrige Phase aus der Extraktion I (17), enthaltend den Alkohol, der bei der Hydrolyse freigesetzt wurde, wird einer weiteren, bevorzugt destillativen Trennung (18) unterzogen. Dabei wird ein Sumpf zur Entsorgung oder weiterer Verarbeitung (24) isoliert. Die Wasserphase (19) wird entweder entsorgt oder in Reaktor III (16) (Leitung (F)) und/oder in die Extraktion I (17) (Leitung (G)) geführt. Der isolierte Alkohol (20), insbesondere das isolierte Methanol im Falle einer MMA-Synthese optional mit kleinen Mengen anderer Stoffe wie z.B. MMA, Wasser etc., wird in Reaktor II geleitet (Leitung D)). Zusammengefasst erfolgt diese Ausführungsform derart, dass nach Prozessschritt e) die weiteren Prozessschritte in der Reihenfolge f), d) und g) durchgeführt werden.

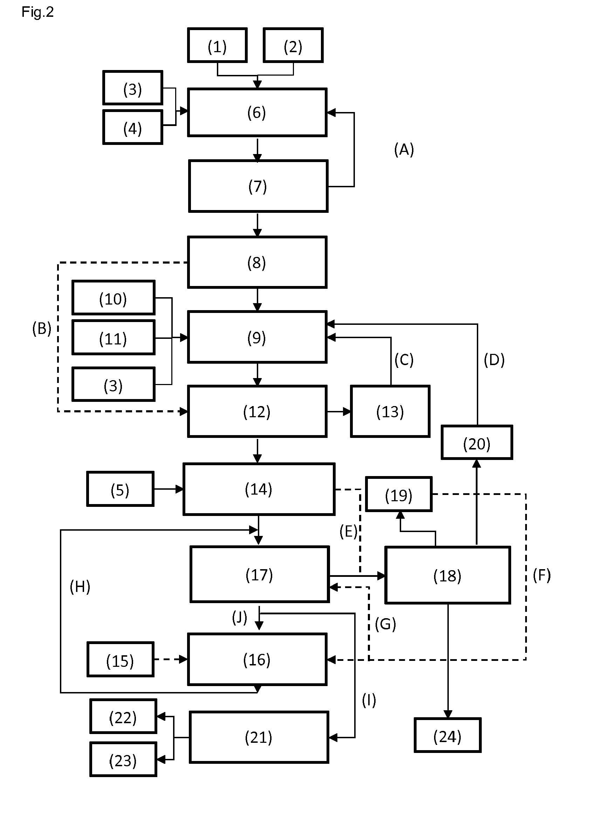

In der zweiten, gleichsam bevorzugten Ausführungsform wird die Alkylmethacrylat enthaltende Zusammensetzung aus Prozessschritt c) zur Durchführung des Prozessschritts d) entgegen der ersten Ausführungsform nicht direkt in den Reaktor III (16), sondern in die Extraktion I (17) geleitet. Anschließend wird die organische Phase aus Prozessschritt d), und damit aus der Extraktion I (17), in einem Teilstrom dem Reaktor III (16) zur Durchführung des Prozessschritts e) (Leitung (J)) und in einem anderen Teilstrom der Trennstufe M (21) zur Durchführung des Prozessschritts g) (Leitung (I)) zugeführt. Ansonsten erfolgt der Aufbau des Gesamtverfahrens analog der ersten Ausführungsform. Ein Vorteil dieser Ausführungsform ist, dass man mittels Schaltung der Ströme (I) und (J) flexibel den Anteil jeweils produzierten Alkylmethacrylats und produzierter Methacrylsäure steuern kann. Die Bildung der Methacrylsäure durch Hydrolyse bzw. Verseifung aus einem Alkylmethacrylat stellt eine Gleichgewichtsreaktion dar. Dabei wird das Gleichgewicht bzw. der Umsatz durch die Prozessparameter wie z.B. Temperatur, Edukt- bzw. Produktenkonzentrationen in Reaktor III gesteuert. Wenn nun das Produktgemisch dieser Umsetzung aus dem Reaktor III (16) zurück in die Extraktion I (17) geführt wird (diesen Strom gibt es auch in Ausführungsform 1), wird dort der freigesetzte Alkohol (wie z.B. Methanol) dem System entzogen. Die jetzt an Alkohol verminderte Mischung wird darauf in dieser Ausführungsform erneut in Reaktor III geleitet, wo eine weitergehende Säurebildung mittels der jetzt bzgl. des Gleichgewichts verschobenen Reaktion erfolgen kann. So ist eine gegenüber Ausführungsform 1 insbesondere gesteigerte Bildung an Methacrylsäure mit dieser Ausführungsform möglich. Eine beispielhafte, schematische Darstellung dieser Ausführungsform findet sich in Fig.2. Zusammengefasst erfolgt diese Ausführungsform derart, dass nach Prozessschritt c) die weiteren Prozessschritte in der Reihenfolge d), e), f) und g), wobei sich durch diese Schaltung eine Kreislaufführung der Prozessschritte d), e) und f) ergibt, durchgeführt werden.

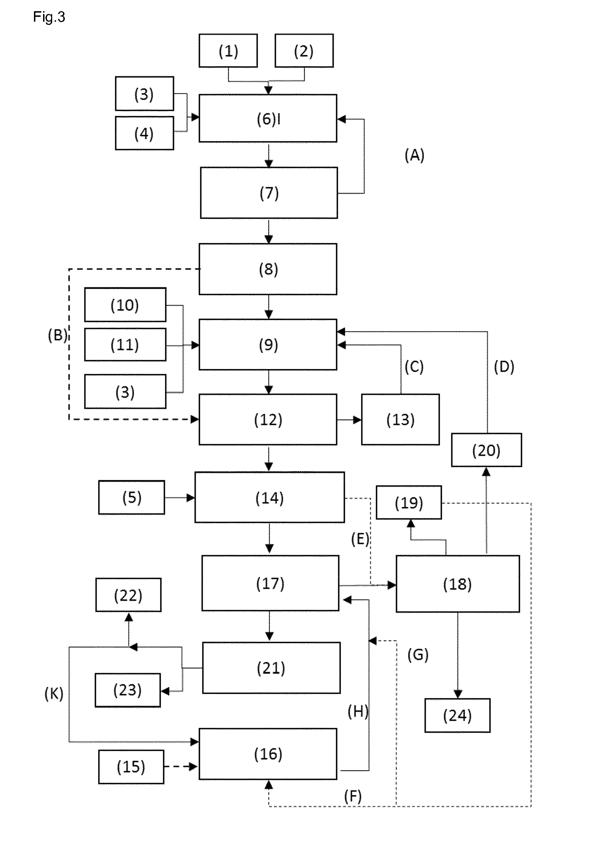

In der dritten, gleichsam bevorzugten Ausführungsform, wie sie beispielshaft in Fig.3 schematisch dargestellt ist, wird die Alkylmethacrylat enthaltende Zusammensetzung aus Prozessschritt c) zur Durchführung des Prozessschritts d) in die Extraktion I (17) geleitet. Anschließend wird die organische Phase aus Prozessschritt d) in die Trennstufe M (21) zur Durchführung des Prozessschritts g) geleitet, wobei anschließend ein Teilstrom (K) aus der Trennstufe M (21) dem Reaktor III (16) zur Durchführung des Prozessschritts e) zugeführt wird. Dabei wird der Teilstrom (K) insbesondere der Alkylmethacrylat-Phase der Trennstufe M (21) entnommen. Das HydrolyseProdukt aus Reaktor III (16) wird schließlich wie in den anderen beiden Ausführungsformen über die Leitung (H) wieder in die Extraktion I (17) überführt. Zusammengefasst erfolgt die Reaktion dementsprechend nach Prozessschritt c) hier in der Reihenfolge d), g), e) und f), wobei diese vier zuletzt genannten Teilschritte zumindest teil- und/oder zeitweise in einer Kreislaufführung betrieben werden.

Optional kann an Prozessschritt c) oder an Prozessschritt d) unabhängig von der eingesetzten Ausführungsform eine weitere Destillation angeschlossen werden, bevor die Alkylmethacrylat-haltige Phase im Prozessschritt e) in Reaktor III geleitet wird. Mittels dieser Destillation wird die Alkylmethacrylat-haltige Phase von hochsiedenden Bestandteilen befreit. Hierzu wird die Alkylmethacrylat-haltige Phase in einer dem Fachmann bekannten Weise in die untere Hälfte einer Destillationskolonne eingebracht. Die Destillationskolonne kann grundsätzlich einer beliebigen, dem Fachmann geeignet erscheinenden Ausführung entsprechen.

Bevorzugt und in der Regel handelt es sich bei dem Alkohol in Prozessschritt b) um Methanol und bei dem Alkylmethacrylat um Methylmethacrylat. Jedoch ist es durchaus auch möglich diese Reaktion mit allen zur Synthese von Alkylmethacrylaten, bzw. di-, tri- oder tetra-Methacrylaten bekannten Alkoholen durchzuführen. Andere Beispiele für solche Alkohole sind dementsprechend Ethanol, Propanol, n-, iso- oder tert-Butanol, 2-Ethylhexanol oder Octanol. Ein Beispiel für diMethacrylate ist Glycol. Auch funktionelle Alkohole, wie zum Beispiel 2-Hydroxy-ethyl-diemethylamin oder mono-Thioglycol können hier verwendet werden.

In einer besonderen Ausführungsform wird die wässrige Phase aus Prozessschritt d) in Wasser, einen Alkohol und einen Abfallstrom getrennt, wobei das Wasser ganz oder teilweise in Reaktor III, der Alkohol ganz oder teilweise in Reaktor II und der Abfallstrom einer Entsorgung zugeleitet werden.

Bezüglich Prozessschritt e) sind diverse Katalysatoren zur Hydrolyse geeignet. Durch die Verwendung von heterogenen Katalysatoren entfällt im Weiteren eine Abtrennung der Katalysatorreste aus der hergestellten Methacrylsäure. Insbesondere sind zur erfindungsgemäßen Hydrolyse mittels heterogener Katalysatoren in Prozessschritt d) Zeolite, Ionenaustauscherharze und amorphen Säurekatalysatoren geeignet. Es können auch Mischungen verschiedener Katalysatoren eingesetzt werden. Als besonders bevorzugt haben sich kationische Ionenaustauscherharze erwiesen. Beispiele für geeignete Katalysatoren sind Ionenaustauscher wie Lewatit K1221, der Fa. Lanxess AG, Lewatit K2629, der Fa. Lanxess AG, Dowex CM-4, der Fa. Dow Chemical, Dowex M-31, der Fa. Dow Chemical, Dowex M-3 MS, der Fa. Dow Chemical, Amberlyst 39 Wet, der Fa. Rohm & Haas, Amberlyst CSP2, der Fa. Rohm & Haas, Amberlyst CSP3, der Fa. Rohm & Haas, DIAION PK208, der Fa. Mitsubishi Chemicals, DIAION PK216, der Fa. Mitsubishi Chemicals, DIAION PK228, der Fa. Mitsubishi Chemicals.

Für die einzelnen Katalysatoren gibt es auch jeweils bevorzugte Ausgestaltungen des Reaktors III, die sich voneinander durchaus unterscheiden können. So kann beispielsweise für Zeolite oder amorphe Säurekatalysatoren insbesondere ein Katalysatorbett in Reaktor III eingesetzt werden. Besonders bevorzugt wird dabei der Reaktor III von oben durchströmt.

Ein solcher Reaktor kann auch für Ionenaustauscherharze zum Einsatz kommen. Alternativ könnte ein Kreislaufreaktor verwendet werden. Dabei wird die Alkylmethacrylat -Phase optional mehrfach durch den Kreislauf geführt werden. Dies wird zum Beispiel dadurch gesteuert, dass die Entnahmegeschwindigkeit aus dem Reaktor deutlich kleiner ist als die Umlaufgeschwindigkeit im Reaktor. Das Masse- oder Volumenverhältnis vom Kreislaufstrom zu dem Feed-Strom, mittels dem die Alkylmethacrylat-haltige Phase in den Reaktor III geleitet wird, beträgt bevorzugt 5 bis 50, besonders bevorzugt 15 bis 30.
Insbesondere bei kationischen Austauscherharzen hat es sich dabei zusätzlich als vorteilhaft erwiesen, wenn die Alkylmethacrylat -Phase vor oder bei der Einleitung in den Reaktor III mit einer Säure, bevorzugt mit Schwefelsäure versetzt wird. Dabei genügen auch kleine Mengen von zum Beispiel 0,01 bis 2 mol% bezogen auf das Alkylmethacrylat.

Aufgrund der in Reaktor II zugegebenen Base, wie zum Beispiel NaOH wird als Nebenprodukt anfallende Methacrylsäure in diesem Fall zum entsprechenden Methacrylatsalz, wie zum Beispiel Natriummethacrylat umgesetzt. Durch die Säurezugabe (14) werden diese Salze wieder in freie Methacrylsäure umgewandelt. Bei der oxidativen Veresterung fallen je nach Prozessführung bis zu 5 Gew%, in der Regel um die 3 Gew% Methacrylsäure als Nebenprodukt an. Wenn diese Säure als Salz auf einen kationischen Ionenaustauscher geleitet wird, verbliebe das Metallion auf dem selbigen, es käme zu einem Effektivitätsverlust und eine nachfolgende Regenerierung des Ionenaustauschers wäre nötig. Durch die Zugabe der starken Säure entfällt ein solcher Verbrauch. Darüber hinaus erfolgt durch das Vorliegen geringen Mengen dieser Säure, insbesondere Schwefelsäure, das lonenaustaucherharz im Betrieb gleichzeitig regeneriert.

Als besonders vorteilhaft hat es sich erwiesen, die Hydrolyse bei einer Temperatur von 50 bis 200 °C, bevorzugt von 90 bis 120 °C und insbesondere zwischen 100 und 110°C durchzuführen. Genauso vorteilhaft hat es sich erwiesen, die Reaktion bei einem Druck von 1,1 bis 10 bar, bevorzugt von 1,5 bis 6 bar durchzuführen. Der Druck wird im Reaktor so eingestellt, dass dieser Druck am Reaktorausgang gemessen wird.

Alternativ kann die Reaktion des Prozessschritts e) in Reaktor III gänzlich unter Vorliegen homogener Katalysatoren, ausgewählt aus der Gruppe der mineralischen und/oder organischen Säuren, bevorzugt Schwefelsäure, Methansulfonsäure oder Toluolulfonsäure bei einer Temperatur von 50 bis 200 °C, bevorzugt von 90 bis 170 °C und bei einem Druck von 1,1 bis 10 bar, bevorzugt von 1,5 bis 6 bar erfolgen.

Die Verweilzeit in Reaktor III hängt insbesondere vom Reaktorvolumen und von den Strömungsgeschwindigkeiten innerhalb des Reaktors ab.

Die Eduktkonzentrationen, Umsatz und Temperatur im Reaktor III können so eingestellt werden, dass das Reaktionsgemisch im Reaktor immer einphasig bleibt. Allerdings ist auch eine Hydrolysereaktion in einem zweiphasigen organisch-wässrigen Gemisch möglich.

Das Alkylmethacrylat /H₂O Molverhältnis des Eduktstromes in Reaktor III liegt bevorzugt zwischen 0,5 und 5, besonders bevorzugt zwischen 1,5 und 3. Dabei es zwar durchaus möglich, zusätzlich geringe Mengen Wasser in den Reaktor III zu leiten. Bevorzugt wird aber der Prozessschritt d) mit der Wassermenge durchgeführt, die in der wässrigen Alkylmethacrylat -Phase aus Prozessschritt c) in Reaktor III geleitet wird. Dieses Wasser entsteht vorher im Prozessschritt b) und somit entfällt bevorzugt der Bedarf nach externer Wasserzufuhr, was sich insgesamt positiv auf die Wirtschaftlichkeit des Verfahrens, insbesondere in energetischer Hinsicht, da ein Aufheizen der Wasserphase entfällt, auswirkt.

Bei der Trennstufe M in Prozessschritt g) handelt es sich bevorzugt um mindestens eine Destillation, eventuell auch um mehrere in Reihe geschaltete Destillationen.

In einer Variante des erfindungsgemäßen Verfahrens, die zur Gewinnung von Alkylmethacrylaten und Methacrylsäure in jeweils benötigten Mengen eingesetzt werden kann, wird vor der Einleitung des jeweiligen Stroms in den Reaktor III entweder ein Teilstrom zur Weiterleitung in eine Aufarbeitung entnommen oder der gesamte Strom zumindest zeitweise vor Reaktor III in die Aufarbeitung umgeleitet, um aus diesem Teilstrom reines Alkymethacrylat zu gewinnen.

Grundsätzlich können rohe Methacrylsäure oder ein rohes Alkylmethacrylat einer weiteren Reinigung unterzogen werden, um zu einem möglichst reinen Produkt zu gelangen. Dieser alternative Prozessschritt zur Reinigung kann beispielsweise einstufig, insbesondere in Form einer mehrstufigen Destillation oder einer Kristallisation, sein. Es hat sich jedoch in vielen Fällen als vorteilhaft herausgestellt, wenn eine solche Reinigung mindestens zwei oder drei Stufen umfasst. Nach einer Vorreinigung durch Abtrennung der hochsiedenden Bestandteile empfiehlt sich eine anschließende Abtrennung der niedersiedenden Bestandteile gefolgt von einer Hauptreinigung.

Zur Aufreinigung der Methacrylsäure, kann die rohe Methacrylsäure zunächst mittels Destillation von niedrigsiedenden Bestandteilen getrennt werden. Die dabei im Kolonnensumpf angereicherte Methacrylsäure kann als Rohmethacrylsäure abgeführt werden. Vorzugsweise wird diese Rohmethacrylsäure in einer weiteren nachgeschalteten Vakuum-Rektifikationskolonne von den enthaltenen Hochsiedern, wie z.B. Stabilisatoren oder Nebenprodukten abgetrennt und über den Kolonnenkopf oder im Seitenstrom als reine Methacrylsäure gewonnen. Die so erhaltene Methacrylsäure weist eine Reinheit von ≥ 99,5 % auf.
Alternativ kann die Methacylsäure auch mittels Kristallisation, mit in der Tendenz noch höheren Reinheiten gereinigt werden. Auch ist es möglich eine Destillations- und eine Kristallisationsstufe miteinander zu kombinieren.

Aufgrund der Polymerisierbarkeit ist es bevorzugt, dass ein oder mehrere Polymerisationsinhibitoren dem Prozess zugegeben werden. Dies betrifft insbesondere alle Prozesstufen des Gesamtprozesses. Polymerisationsinhibitoren, wie beispielsweise Hydrochinone, Hydrochinonether, wie Hydrochinonmonomethylether oder Di-tert-butylbrenzcatechin, Phenothiazin, N,N'-(Diphenyl)-p-phenylendiamin, 4 Hydroxy-2,2,6,6-tetramethylpiperidin-1-oxyl, p-Phenylendiamin, Methylenblau oder sterisch gehinderte Phenole, sind in der Fachwelt weithin bekannt. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der Polymerisation auftretenden freien Radikale wirken. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Das erfindungsgemäße Verfahren wird derart betrieben, dass der molare Anteil der isolierten Methacrylsäure an der Summe aus isolierter Methacrylsäure und isolierter Alkylmethacrylat zwischen 0,02 und 1 liegt. Bevorzugt wird das erfindungsgemäße Verfahren derart betrieben, dass dieses Verhältnis zwischen 0,05 und 0,5 und besonders bevorzugt zwischen 0,1 und 0,3 liegt. Die Metharcylsäure wird in dem erfindungsgemäßen Verfahren in den Prozessschritten b) und insbesondere e) gebildet. Während die Methacrylsäure-Bildung in Prozessschritt b) als Nebenprodukt vergleichsweise gering ist, kann die Bildung der Methacrylsäure in Prozessschritt e) in einem weiten Bereich gesteuert werden, indem man Einflussfaktoren wie die Ausgestaltung des Prozessschritts e), insbesondere in Bezug auf die Anzahl der Durchläufe der Prozessschritte d) und e), die Steuerung der Verhältnisse der Produktströme, entnehmend das Alkylmethacrylat bzw. in den Reaktor III führend, sowie die Zusammensetzung in Reaktor III, variiert. Im einfachsten Fall kann man beispielsweise durch eine Erhöhung des Wasseranteils in Reaktor III die Bildung der Methacrylsäure, z.B. durch Beeinflussung des Gleichgewichts und der Reaktionsgeschwindigkeit, steuern. Aufgrund der Gleichgewichtsreaktion ist ein besonders hoher Anteil zu bildender Methacrylsäure von mehr als 50 Mol% energetisch eher nachteilig.

Neben dem erfindungsgemäßen Verfahren ist auch eine dazu einsetzbare Anlage zur Herstellung von Alkylmethacrylaten und von Methacrylsäure Bestandteil der vorliegenden Erfindung. Eine solche Anlage kann insbesondere dadurch gekennzeichnet, dass diese mindestens folgende Vorrichtungen aufweist:
a) einen Reaktor I zur Synthese von Methacrolein,
b) einen Reaktor II zur oxidativen Veresterung von Methacrolein zu einem Alkylmethacrylat in Gegenwart von einem Alkohol und Sauerstoff,
c) eine erste Destillationskolonne zur Destillation des Reaktoraustrags aus Reaktor II, eine optionale Behandlung mit einer Säure und optional anschließende Phasentrennung der Alkylmethacrylat-haltigen Phase aus der Destillation,
d) eine Extraktion I zur Trennung Alkylmethacrylat und optional Methacrylsäure enthaltenden Zusammensetzung unter Zuführen von Wasser in eine organische und eine wässrige Phase
e) einen Reaktor III zur teilweisen Hydrolyse des Alkylmethacrylats zu Methacrylsäure
f) eine Rohrleitung aus Reaktor III, die direkt oder indirekt in die Extraktion I führt und
g) mindestens eine zweite Destillationskolonne und/oder eine Extraktion zur Trennung des Alkylmethacrylats von der Methacrylsäure aufweist.

### Beispiele

### Beispiel 1

Der Gesamtenergieverbrauch des Herstellverfahrens von MMA aus Methacrolein (laut Fig.1 aber ohne Reaktor III) wurde ermittelt und verglichen mit den entsprechenden Werten für einen erfindungsgemäßen Verfahren zur Herstellung von MMA und MAS gemäß Fig.1bis Fig. 3:

**Tabelle 1**

| Beispiel | Verfahren | MMA [kt/a] | MAS [kt/a] | MMA eq. [kt/a] | Energieverbrauch [t Dampf/t MMA eq.] |
|---|---|---|---|---|---|
| Vergleichsbeispiel | (ohne Rkt III) | 143 | 3 | 146 | 5,13 |
| Beispiel 1 | gem. Fig. 1 | 126 | 18 | 144 | 4,97 |
| Beispiel 2 | gem. Fig. 2 | 98 | 43 | 141 | 4,90 |
| Beispiel 3 | gem. Fig. 3 | 99 | 43 | 142 | 6,00 |

Wie man aus Tabelle 1 erkennen kann, lassen sich bei allen erfindungsgemäßen Ausführungsbeispielen relativ hohe Mengen an MAS zusätzlich zu MMA produzieren. Der Gesamtenergiebedarf bleibt dabei ähnlich dem Verfahren ohne MAS Produktion.

### Bezugszeichenliste

In Fig. 1 ist eine Prozessführung gemäß Ausführungsform 1 dargestellt. Diese Ausführungsform ist derart gestaltet, dass nach Prozessschritt e) die weiteren Prozessschritte in der Reihenfolge f), d) und g) durchgeführt werden.

In Fig. 2 ist eine Prozessführung gemäß Ausführungsform 2 dargestellt. Diese Ausführungsform ist derart gestaltet, dass nach Prozessschritt c) die weiteren Prozessschritte in der Reihenfolge d), e), f) und g), wobei sich durch diese Schaltung eine Kreislaufführung der Prozessschritte d), e) und f) ergibt, durchgeführt werden.

In Fig. 3 ist eine Prozessführung gemäß Ausführungsform 3 dargestellt. Hier erfolgt die Reaktion nach Prozessschritt c) in der Reihenfolge d), g), e) und f), wobei Prozessschritte d), g), e) und f) zumindest teil- und/oder zeitweise in einer Kreislaufführung betrieben werden.
(1) Formaldehyd-Feed
(2) Propionaldehyd-Feed
(3) Zuleitung einer Base
(4) Zuleitung einer Säure I
(5) Zuleitung einer Säure (II)
(6) Reaktor I (Synthese von Methacrolein; Prozessschritt a)
(7) Katalysatorabtrennung
(8) Isolierung Methacrolein
(9) Reaktor II (oxidative Veresterung; Prozessschritt b)
(10) Alkohol-Feed (z.B. Methanol)
(11) Sauerstoff-bzw. Luft-Zuleitung
(12) Abtrennung von Methacrolein und Alkohol (z.B. Methanol) (Prozessschritt c)
(13) Isoliertes Methacrolein/Alkohol Gemisch
(14) Versetzen mit Säure und optionale Phasentrennung
(15) Optionale Wasser und Säure (II) Zugabe
(16) Reaktor III (Hydrolyse zu Methacrylsäure; Prozessschritt e)
(17) Extraktion I (Prozessschritt d)
(18) Trennung des isolierten Alkohol/H₂O-Gemischs (aus Prozessschritt d)
(19) Abgeführtes H₂O (zur Zurückführung oder Entsorgung)
(20) Isolierter Alkohol (z.B. Methanol)
(21) Trennstufe M (Trennung Alkylmethacrylat und Methacrylsäure; Prozessschritt g)
(22) Isoliertes Alkylmethacrylat zur weiteren Reinigung
(23) Isolierte Methacrylsäure zur weiteren Reinigung
(24) Entsorgung

Besondere Leitungen:
(A) Katalysatorrückführung in Reaktor I
(B) Optionale Überführung des Methacrolein-Stroms in die Methacrolein Reinigung (12)
(C) Rückführung abgetrenntes Methacrolein (mit Methanol) in (9)
(D) Rückführung Methanol in (9)
(E) Optionale Überführung der wässrigen Phase aus (14) in (18)
(F) Optionale Überführung des Wassers aus (18) in (16)
(G) Optionale Überführung des Wassers aus (18) in (17)
(H) Überführung Produktstrom aus (16) in (17) (Prozessschritt f)

Nur in Fig.2:
(I) Teilstrom aus (17) in (21)
(J) Teilstrom aus (17) in (16)

Nur in Fig.3:
(K) Teilstrom der Alkylmethacrylat-Phase (22) aus (21) in (16)

## Patentansprüche

1. Verfahren zur Herstellung von Alkylmethacrylaten und Methacrylsäure, aufweisend folgende Prozessschritte:
a) Synthese von Methacrolein in einem Reaktor I,
b) Oxidative Veresterung von Methacrolein mit einem Alkohol und Sauerstoff zu einem Alkylmethacrylat in einem Reaktor II und
e) Umsetzung mindestens eines Teils des Alkylmethacrylats mit Wasser zu Methacrylsäure in einem Reaktor III.

2. Verfahren gemäß Anspruch 1, aufweisend folgende Prozessschritte:
a) Synthese von Methacrolein in einem Reaktor I,
b) Oxidative Veresterung von Methacrolein mit einem Alkohol und Sauerstoff zu einem Alkylmethacrylat in einem Reaktor II,
c) Abtrennen überschüssigen Methacroleins und zumindest teilweises Abtrennen des Alkohols, anschließende optionale Behandlung mit einer Säure und optionale Phasentrennung der Alkylmethacrylat enthaltenden Zusammensetzung,
d) Trennen der Alkylmethacrylat und optional Methacrylsäure enthaltenden Zusammensetzung unter Zuführen von Wasser als eine organische Phase von einer wässrigen Phase in einer Extraktion I,
e) Umsetzung mindestens eines Teils des Alkylmethacrylats mit Wasser zu Methacrylsäure in einem Reaktor III,
f) Überführen der Zusammensetzung, enthaltend Methacrylsäure und Alkylmethacrylate aus Prozessschritt e) in die Extraktion I und
g) optimale Trennung der Methacrylsäure von dem Alkylmethacrylat in einer Trennstufe M.

3. Verfahren gemäß Anspruch 1 oder 2, **dadurch gekennzeichnet, dass** in Prozessschritt b) gebildetes Reaktionswasser zwischen den Prozessschritten b) und e) von dem Alkylmethacrylat abgetrennt und in Prozessschritt e) dem Reaktor III zur Hydrolyse ganz oder teilweise wieder zugeführt wird.

4. Verfahren gemäß Anspruch 1, 2 oder 3, **dadurch gekennzeichnet, dass** der in Prozessschritt e) gebildete Alkohol ganz oder teilweise abgetrennt und in Prozessschritt b) dem Reaktor II zur oxidativen Veresterung ganz oder teilweise wieder zugeführt wird.

5. Verfahren gemäß Anspruch 2, 3 oder 4, **dadurch gekennzeichnet, dass** die Alkylmethacrylat enthaltende Zusammensetzung aus Prozessschritt c) zur Durchführung des Prozessschritts e) in den Reaktor III geleitet wird und nach Prozessschritt e) nacheinander die Prozessschritte f), d) und g) durchgeführt werden.

6. Verfahren gemäß Anspruch 2, 3 oder 4, **dadurch gekennzeichnet, dass** die Alkylmethacrylat enthaltende Zusammensetzung aus Prozessschritt c) zur Durchführung des Prozessschritts d) in die Extraktion I geleitet wird, die organische Phase aus Prozessschritt d) anschließend in einem Teilstrom dem Reaktor III zur Durchführung des Prozessschritts e) und in einem anderen Teilstrom der Trennstufe M zur Durchführung des Prozessschritts g) zugeführt wird.

7. Verfahren gemäß Anspruch 2, 3 oder 4, **dadurch gekennzeichnet, dass** die Alkylmethacrylat enthaltende Zusammensetzung aus Prozessschritt c) zur Durchführung des Prozessschritts d) in die Extraktion I geleitet wird und die organische Phase aus Prozessschritt d) anschließend in die Trennstufe M zur Durchführung des Prozessschritts g) geleitet wird, wobei anschließend ein Teilstrom der Alkylmethacrylat-Phase aus der Trennstufe M dem Reaktor III zur Durchführung des Prozessschritts e) zugeführt wird.

8. Verfahren gemäß Anspruch 5, 6 oder 7, **dadurch gekennzeichnet, dass** die wässrige Phase aus Prozessschritt d) in Wasser, einen Alkohol und einen Abfallstrom getrennt wird, wobei das Wasser ganz oder teilweise in Reaktor III, der Alkohol ganz oder teilweise in Reaktor II und der Abfallstrom einer Entsorgung zugeleitet wird.

9. Verfahren gemäß mindestens einem der Ansprüche 2 bis 8, **dadurch gekennzeichnet, dass** das Methacrolein in Prozessschritt a) aus Propionaldehyd und Formaldehyd über eine Mannich Kondensation hergestellt wird, und dass das in Prozessschritt c) abgetrennte Methacrolein und der Alkohol in den Reaktor II zurückgeführt werden.

10. Verfahren gemäß mindestens einem der Ansprüche 2 bis 8, **dadurch gekennzeichnet, dass** es sich bei der Trennstufe M in Prozessschritt g) um mindestens eine Destillation handelt.

11. Verfahren gemäß mindestens einem der Ansprüche 1 bis 10, **dadurch gekennzeichnet, dass** die Reaktion des Prozessschritts e) in Reaktor III unter Vorliegen heterogener Katalysatoren ausgewählt aus der Gruppe der Zeolite, Ionenaustauscherharze und amorphen Säurekatalysatoren, bevorzugt kationische Ionenaustauscherharze bei einer Temperatur von 50 bis 200 °C, bevorzugt von 90 bis 120 °C und bei einem Druck von 1,1 bis 10 bar, bevorzugt von 1,5 bis 6 bar erfolgt.

12. Verfahren gemäß mindestens einem der Ansprüche 1 bis 10, **dadurch gekennzeichnet, dass** die Reaktion des Prozessschritts e) in Reaktor III unter Vorliegen homogener Katalysatoren ausgewählt aus der Gruppe der mineralischen und/oder organischen Säuren, bevorzugt Schwefelsäure, Methansulfonsäure oder Toluolulfonsäure bei einer Temperatur von 50 bis 200 °C, bevorzugt von 90 bis 170 °C und bei einem Druck von 1,1 bis 10 bar, bevorzugt von 1,5 bis 6 bar erfolgt.

13. Verfahren gemäß mindestens einem der Ansprüche 1 bis 12, **dadurch gekennzeichnet, dass** es sich bei dem Alkohol um Methanol und bei dem Alkylmethacrylat um MMA handelt.

14. Verfahren gemäß mindestens einem der Ansprüche 1 bis 13, **dadurch gekennzeichnet, dass** vor der Einleitung des jeweiligen Stroms in den Reaktor III zur Gewinnung von reinem Alkymethacrylat entweder ein Teilstrom zur Weiterleitung in eine Aufarbeitung entnommen wird oder der gesamte Strom zumindest zeitweise vor Reaktor III in die Aufarbeitung umgeleitet wird.

15. Verfahren gemäß mindestens einem der Ansprüche 1 bis 14, **dadurch gekennzeichnet, dass** das Verfahren derart betrieben wird, dass der molare Anteil der isolierten Methacrylsäure an der Summe aus isolierter Methacrylsäure und isolierter Alkylmethacrylat zwischen 0,02 und 1, bevorzugt zwischen 0,05 und 0,5 und besonders bevorzugt zwischen 0,1 und 0,3 liegt.

16. Anlage zur Herstellung von Alkylmethacrylaten und von Methacrylsäure, **dadurch gekennzeichnet, dass** die Anlage mindestens
a) einen Reaktor I zur Synthese von Methacrolein,
b) einen Reaktor II zur oxidativen Veresterung von Methacrolein zu einem Alkylmethacrylat in Gegenwart von einem Alkohol und Sauerstoff,
c) eine Destillationskolonne zur Destillation des Reaktoraustrags aus Reaktor II, eine optionale Behandlung mit einer Säure und optional anschließende Phasentrennung der Alkylmethacrylat-haltigen Phase aus der Destillation,
d) eine Extraktion I zur Trennung Alkylmethacrylat und optional Methacrylsäure enthaltenden Zusammensetzung unter Zuführen von Wasser in eine organische und eine wässrige Phase
e) einen Reaktor III zur teilweisen Hydrolyse des Alkylmethacrylats zu Methacrylsäure
f) eine Rohrleitung aus Reaktor III, die direkt oder indirekt in die Extraktion I führt und
g) mindestens eine Destillationskolonne zur Trennung des Alkylmethacrylats von der Methacrylsäure
aufweist.
